# EUROPEAN PATENT SPECIFICATION

(11) **EP 2 945 574 B1**
(45) Date of publication and mention of the grant of the patent: **18.12.2019**
(21) Application number: 14800405.4
(22) Date of filing: 16.01.2014
(51) Int. Cl.: A61F 2/44, A61F 2/46, A61B 17/80, A61B 17/17, A61B 17/70, A61F 2/30

(54) **SPINAL PLATE SELECTION AND POSITIONING SYSTEM**
SPINALPLATTENAUSWAHL- UND POSITIONIERUNGSSYSTEM
SYSTÈME DE SÉLECTION ET DE POSITIONNEMENT DE PLAQUE SPINALE

(30) Priority: 16.01.2013 US 201361753420 P
(43) Date of publication of application: 25.11.2015
(73) Proprietor: Retrospine Pty Ltd., Sydney, New South Wales 2747 (AU)
(72) Inventor: SEEX, Kevin, Sydney, New South Wales 2747 (AU)
(74) Representative: Walker, Ross Thomson
(86) International application number: PCT/IB2014/001773
(87) International publication number: WO 2014/188280

(56) References cited:
- EP-A1- 2 352 462
- WO-A1-2010/107692
- WO-A1-2013/036687
- WO-A2-2008/034140
- WO-A2-2008/070863
- AU-A1- 2012 251 944
- US-A1- 2002 147 450
- US-A1- 2005 159 813
- US-A1- 2006 189 997
- US-A1- 2007 270 965
- US-A1- 2008 294 262
- US-A1- 2009 012 529
- US-A1- 2009 024 132
- US-A1- 2009 024 132
- US-A1- 2010 228 297
- US-A1- 2010 228 297
- US-A1- 2011 190 892
- US-A1- 2012 232 599
- US-B1- 8 840 667
- US-B2- 7 172 627

## Description

### BACKGROUND

### 1. The Field of the Present Disclosure.

The present disclosure relates generally to medical devices, and more particularly, but not necessarily entirely, to medical implants and instrumentation utilized in spinal-fusion surgical procedures.

### 2. Description of Related Art.

Spinal fusion surgery is often performed to reduce pain caused by aging or injury. Spinal fusion surgery typically involves fusing two or more vertebrae in the spinal column. Fusion of the vertebrae may be accomplished using a bone fixation device, such as an interbody plate. In particular, rigid interbody plates may be secured to the vertebrae. The interbody plates may stabilize the spinal column. Fasteners, such as bone screws, may be utilized to secure the interbody plates to the vertebrae. For this reason, interbody plates are generally rigid but may have some adjustability for sizing purposes. Rods may sometimes also be utilized to provide greater stabilization.

It is further known to use an interbody cage in conjunction with a spinal fusion procedure. In particular, an interbody cage may be implanted by press fit into the disc space between two adjacent vertebrae. (This, of course, may first require removal of the patient's natural disc.) In this regard, the use of an interbody cage has been found to reduce postoperative discomfort and pain. Interbody cages may take a wide variety of forms. For example, interbody cages may be formed from alloys or plastic. In some instances, interbody cages are packed with autologous bone material in order to promote fusion. That is, this supplementary bone material, such as an allograft or an autograft, may be used in conjunction with the patient's natural bone growth process to fuse the vertebrae. In other instances, interbody cages may be made entirely of, or partly of, either a human graft (allograft or autograft) or an animal graft (xenograft).

An interbody plate and an interbody cage may be utilized together. In this instance, the interbody plate may extend over a disc space containing the interbody cage. The interbody plate may then be secured to the two vertebrae defining the disc space using fasteners. An interbody plate is ideally positioned parallel to the longitudinal axis of the spine and symmetrically over the disc space. In practice, however, proper alignment and positioning of the interbody cage may be difficult to accomplish due to (i) narrow wounds which obscure surgical landmarks; (ii) blood or other tissue may obscure landmarks; (iii) the interbody plate itself may obscure landmarks; (iv) drill guides that are used to drill pilot holes may further obscure landmarks; and (v) the interbody plate may move prior to being secured without the surgeon being aware.

In the past, some techniques have been developed in an attempt to properly align and position interbody plates and interbody cages. One technique includes the use of a trial cage connected to a drill guide. In particular, the trial cage is temporarily installed into the disc space. The attached drill guide is then utilized to drill pilot holes in the adjacent vertebrae. The trial cage and drill guide are then removed and the interbody cage is implanted into the disc space. The plate is then positioned and fastened to the vertebrae using the pilot holes. This technique, however, has limitations. One limitation is that there is no assurance that the trial cage and the actual interbody cage are located in the same position. Another limitation is that there is no assurance that the interbody plate is positioned correctly with respect to the pilot holes. A further limitation is that the positioning of the interbody cage relative to the interbody plate is not controlled.

Another attempt to properly align and position interbody plates and interbody cages is taught by Fraser et al. (U.S. Patent Publication No. 2011/004253). Fraser teaches the use of an interbodyplate that includes integral mating elements that are adapted to slidably engage an interbody cage. One draw back to Fraser's teachings is that there is no assurance that the interbody plate is centered over the interbody cage or that the cage is properly positioned in the disc space prior to the plate being secured.

Despite the advantages of known alignment and positioning techniques, improvements are still being sought. For example, many of the prior art devices such as U.S. Patent Publications No. 2101/228297 and US 2007/0270965, cannot assure that an interbody plate and interbody cage are ideally positioned. The prior art is thus characterized by several disadvantages that are addressed by the present disclosure. The present disclosure minimizes, and in some aspects eliminates, the above-mentioned failures, and other problems, by utilizing the methods and structural features described herein.

The features and advantages of the present disclosure will be set forth in the description which follows, and in part will be apparent from the description, or may be learned by the practice of the present disclosure without undue experimentation. The features and advantages of the present disclosure may be realized and obtained by means of the instruments and combinations particularly pointed out in the appended claims.

### BRIEF DESCRIPTION OF THE DRAWINGS

The features and advantages of the disclosure will become apparent from a consideration of the subsequent detailed description presented in connection with the accompanying drawings in which:
FIG. 1 is a perspective view of an interbody cage according to an embodiment of the present disclosure;
FIG. 2 is a perspective view of a holding rod according to an embodiment of the present disclosure;
FIG. 3 is a perspective view of an interbody plate according to an embodiment of the present disclosure;
FIG. 4 is a perspective view of a guide member according to an embodiment of the present disclosure;
FIG. 5 is a perspective view of a drill guide according to an embodiment of the present disclosure;
FIG. 6 is another perspective view of the drill guide shown in FIG. 5 according to an embodiment of the present disclosure;
FIG. 7 is a perspective view of another drill according to an embodiment of the present disclosure;
FIG. 8 is an exploded view of an assembly according to an embodiment of the present disclosure;
FIG. 9 is a partially exploded view of the assembly shown in FIG. 8 according to an embodiment of the present disclosure;
FIG. 10 depicts a procedure for installing an interbody cage in a disc space according to an embodiment of the present disclosure;
FIG. 11 depicts a procedure for drilling pilot holes and installing plate fasteners in a vertebra according to an embodiment of the present disclosure;
FIG. 12 depicts a procedure for drilling pilot holes and installing plate fasteners in a vertebra according to an embodiment of the present disclosure;
FIG. 13 depicts a procedure for removing a drill guide according to an embodiment of the present disclosure;
FIG. 14 depicts an installed interbody plate and interbody cage according to an embodiment of the present disclosure;
FIG. 15 depicts a pair of installed interbody plates and interbody cages according to an embodiment of the present disclosure;
FIGS. 16-17 depict an alternative embodiment of a drill guide, having a hollow handle;
FIGS. 18-19 depict section 502A of the drill guide of FIGS. 16-17, and detail shown thereby.
FIG. 20 illustrates further detail of the matter shown in FIGS. 16-19.
FIG. 21 illustrates an alternative embodiment of a spinal positioning plate in association with a cage;
FIG. 22 shows the cage of FIG. 21;
FIG. 23 shows the plate of FIG. 21.

### DETAILED DESCRIPTION

For the purposes of promoting an understanding of the principles in accordance with the disclosure, reference will now be made to the embodiments illustrated in the drawings and specific language will be used to describe the same. It will nevertheless be understood that no limitation of the scope of the disclosure is thereby intended. Any alterations and further modifications of the inventive features illustrated herein, and any additional applications of the principles of the disclosure as illustrated herein, which would normally occur to one skilled in the relevant art and having possession of this disclosure, are to be considered within the scope of the disclosure claimed.

In describing and claiming the present disclosure, the following terminology will be used in accordance with the definitions set out below. It must be noted that, as used in this specification and the appended claims, the singular forms "a," "an," and "the" include plural referents unless the context clearly dictates otherwise. As used herein, the terms "comprising," "including," "containing," "characterized by," and grammatical equivalents thereof are inclusive or open-ended terms that do not exclude additional, unrecited elements or method steps.

Applicant has discovered a spinal plate selection and positioning system for use in a spinal fusion surgery. In an embodiment, the system may include a guide member removably attached to an interbody cage by a holding rod. After the interbody cage has been installed into a disc space defined between a first vertebra and a second vertebra using the holding rod, the guide member may be utilized to align and position an interbody plate over the disc space. The guide member may center the interbody plate over the interbody cage and also prevent the interbody plate from rotating about a longitudinal axis of the guide member. Thus, the guide member may align and position the interbody plate with reference to the interbody cage since the guide member and interbody cage are coupled.

A drill guide may then also be aligned and positioned over the disc space using the guide member. Pilot holes may then be drilled in the vertebrae defining the disc space using the drill guide. Using the drill guide, fasteners may be installed to secure the interbody plate to the vertebrae. The drill guide may then be removed from the guide member and the guide member may be detached from the interbody cage, now secured in the disc space beneath the interbody plate.

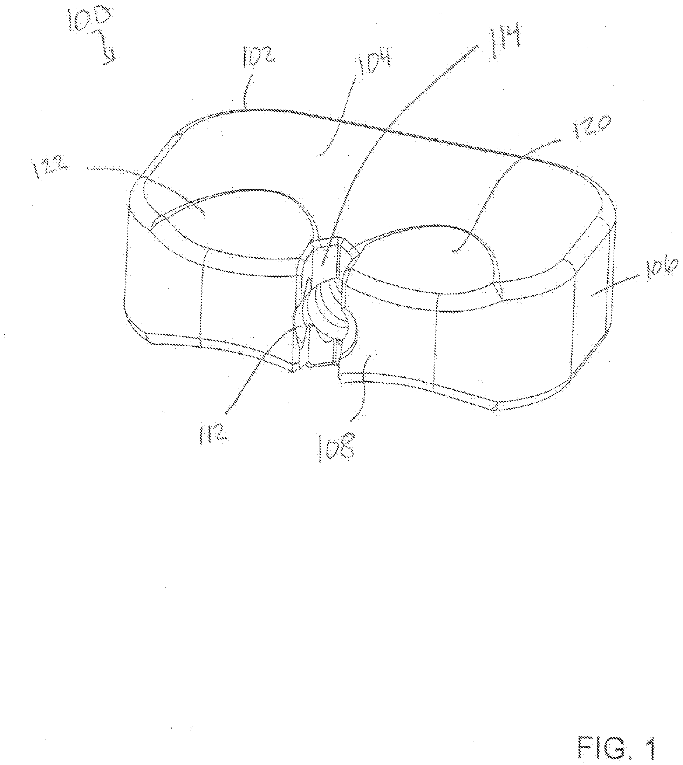

Referring now to FIG. 1, there is depicted an exemplary interbody cage 100 pursuant to an embodiment of the present disclosure. The interbody cage 100 may include a body member 102. The body member 102 may have a superior surface 104 and an inferior surface 105 (not explicitly visible in FIG. 1, but understood to be opposite of the superior surface 104). A sidewall 106 may circumscribe the body member 102. The sidewall 106 may extend between the superior surface 104 and the inferior surface 105. The sidewall 106 may define, for the body member 102, a proximal surface 108 and a distal surface 110 (not explicitly visible in FIG. 1, but understood to be opposite of the proximal surface 108).

A threaded bore 112 maybe formed in the proximal surface 108 of the sidewall 106. As will be explained in more detail hereinafter, the threaded bore 112 may engage a threaded end of a holding rod that is utilized to position and implant the interbody cage 100 in a disc space defined between two adjacent vertebrae. A key slot 114 may also be formed in the proximal surface 108 of the sidewall 106. The key slot 114 may extend from the superior surface 104 to the inferior surface 105 of the body member 102. In an embodiment, the key slot 114 may pass through the threaded bore 112. As will be explained in more detail hereinafter, the key slot 114 may engage key members extending from a guide member.

Formed in the superior surface 104 of the body member 102 maybe grooves or cutouts 120 and 122. As will be explained in detail hereinafter, the grooves 120 and 122 may form reliefs in the superior surface 104 to allow passage of fasteners utilized to secure an interbody plate to vertebrae in a spinal column. The grooves 120 and 122 may extend from the proximal surface 108 of the sidewall 106 toward the distal surface 110 of the sidewall 106. The grooves 120 and 122 may taper upwards, in the superior direction, as they extend toward the distal surface 110 such that they taper out.

One of the grooves 120 and 122 maybe disposed on either side of the threaded bore 112. It will be appreciated that the inferior surface 105 of the body member may contain grooves similar in size, shape and location to the grooves 120 and 122 in the superior surface 104. In an embodiment, the grooves in the inferior surface 105 may differ in size, shape, and location to the grooves 120 and 122 in the superior surface 104.

Although the body member 102 is depicted as a solid member, it will be appreciated that the body member 102 may take a wide variety of configurations according to embodiments of the present disclosure. In an embodiment, the body member 102 may comprise a hollow interior portion for receiving a bone graft. Further, the body member 102 may be formed from a wide range of biocompatible materials as is known to one having ordinary skill.

Pursuant to an embodiment of the present disclosure, the present disclosure may provide a plurality of interbody cages of varying height, i.e., the distance between the superior surface 104 and the inferior surface 105, to accommodate disc spaces of varying sizes. In an embodiment, a surgeon may trial fit multiple interbody cages until the right fit is obtained.

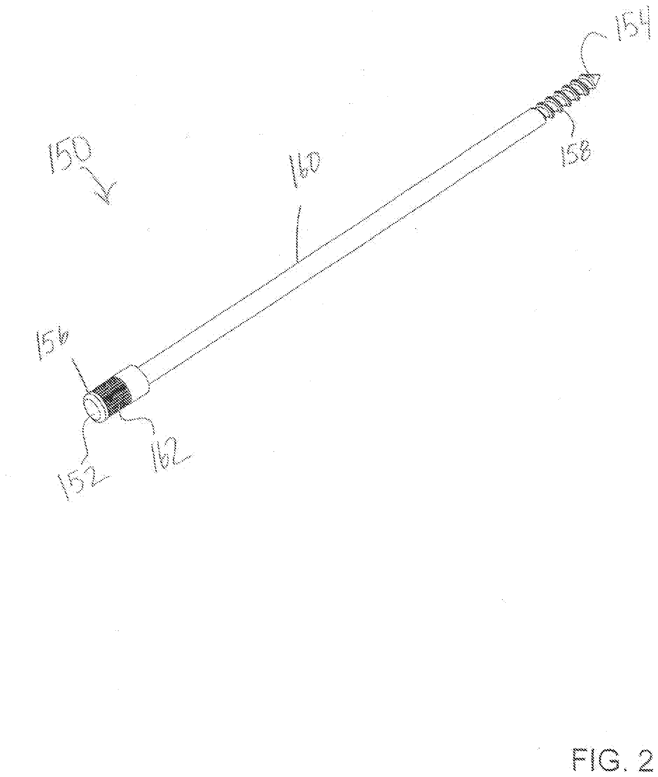

Referring now to FIG. 2, there is depicted a holding rod 150 pursuant to an embodiment of the present disclosure. The holding rod 150 may extend from a proximal end 152 to a distal end 154. A knob 156 may be located at the proximal end 152 of the holding rod 150. The distal end 154 may include a plurality of threads 158. A shaft 160 may extend between the proximal end 152 and the distal end 154. The knob 156 may include grip enhancing means 162, such as serrations. In an embodiment, the threads 158 on the distal end 154 of the holding rod 150 are configured and adapted to engage the threaded bore 112 of the interbody cage 100 (see FIG. 1). It will be appreciated that the holding rod 150 may be utilized to facilitate the installation of the interbody cage 100 into a disc space between two adjacent vertebrae.

The present disclosure further contemplates other means of securing the holding rod 150 to the interbody cage 100. In an embodiment, the holding rod 150 may engage the interbody cage 100 by a snap or press fit. In an embodiment, the holding rod 150 may include an expandable head, activated proximally, that engages a receiving portion in the interbody cage 100 when the head is expanded. In an embodiment, the holding rod 150 may engage the interbody cage 100 using a twist-lock mechanism.

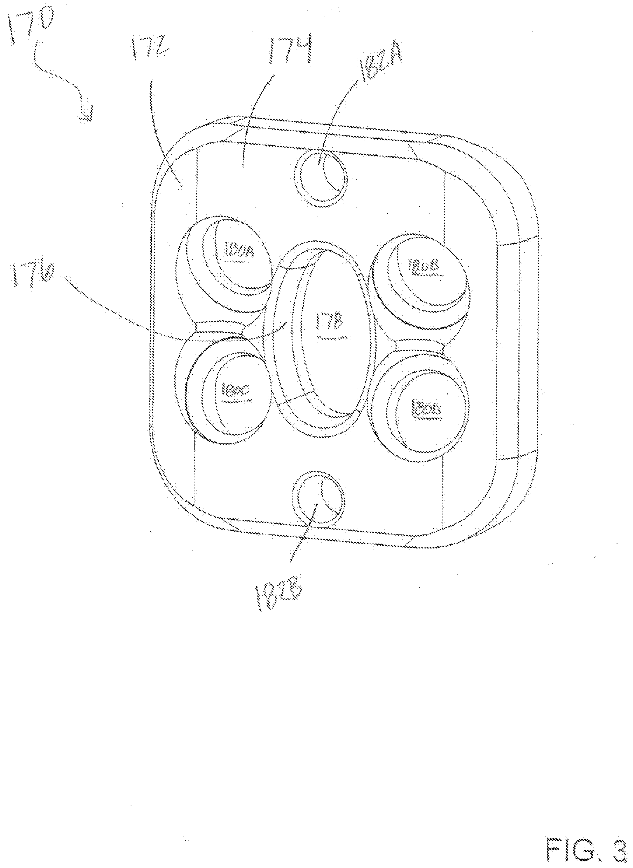

Referring now to FIG. 3, there is depicted an interbody plate 170 pursuant to an embodiment of the present disclosure. The plate 170 may include a body member 172 according to an embodiment of the present disclosure. The body member 172 may include a proximal end 174 and a distal end 175 (not explicitly visible in FIG. 3, but the distal end 175 is understood to be on the opposite side of the interbody plate 170 from the proximal end 174). In an embodiment, the body member 172 of the plate 170 is substantially rectangular in shape.

An inner surface 176 of the body member 172 may define a guide hole 178. In an embodiment, the guide hole 178 may be located in the center of the body member 172. The guide hole 178 may extend from the proximal end 174 to the distal end 175. In an embodiment, the guide hole 178 may be noncircular. In an embodiment, the guide hole 178 may be oval.

The body member 172 may further comprise fastener holes 180A, 180B, 180C and 180D. Holes 180A and 180B may define a first set of holes and holes 180C and 180D may define a second set of holes. The first set of holes 180A, 180B may allow fasteners (not explicitly shown) to secure the plate 170 to a first vertebra (not explicitly shown) and the second set of holes 180C, 180D may allow fasteners (not explicitly shown) to secure the plate 170 to a second vertebra (not explicitly shown). One of the first set of holes 180A, 180B may be disposed on either side of the guide hole 178. One of the second set of holes 180C, 180D may be disposed on either side of the guide hole 178. The first set of holes 180A, 180B may be angled. The second set of holes 180C, 180D may be angled.

The body member 172 may further comprise a pair of guide holes 182A and 182B. The guide holes 182A and 182B may facilitate the positioning of a drill guide over the holes 180A-180D as will be explained in detail hereinafter.

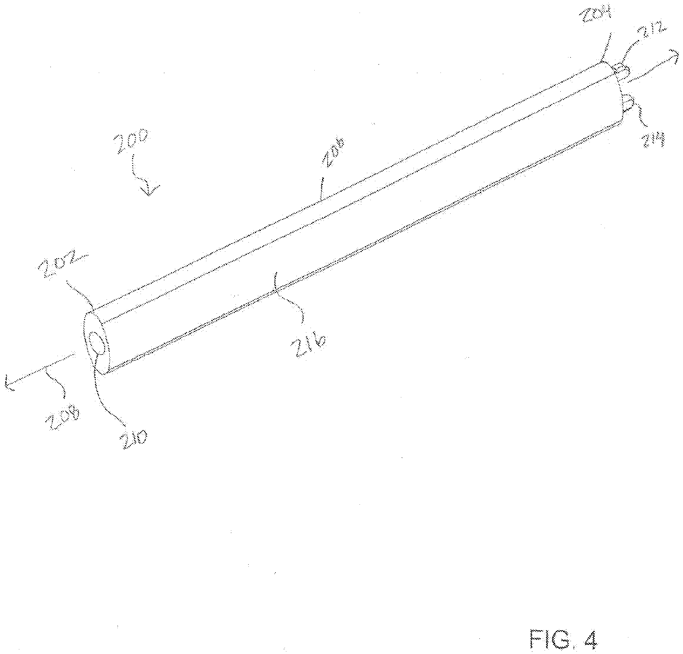

Referring now to FIG. 4, there is depicted an elongated guide member 200 pursuant to an embodiment of the present disclosure. The guide member 200 may have a proximal end 202 and a distal end 204. A body portion 206 of the guide member 200 may extend between the proximal end 202 and the distal end 204. The body portion 206 may extend along a longitudinal axis 208. A cannulation 210 may extend from the proximal end 202 to the distal end 204.

In an embodiment, the guide member 200 may include key members 212 and 214 extending from the distal end 204. The key members 212 and 214 are configured and adapted to engage the key slot 114 of the interbody cage 100 (see FIG. 1) as will be explained in more detail hereinafter. It will be appreciated that the use of the key members 212 and 214, and the key slot 114, prevent the elongated guide member 200 from rotating about its longitudinal axis 208 when removably secured to the interbody cage 100.

In an embodiment, the cannulation 210 is configured and adapted to allow the shaft 160 of the holder rod 150 (see FIG. 2) to be inserted into, and pass through, the guide member 200 According to the invention, a cross section of the guide member 200 in a plane perpendicular to its longitudinal axis 208 is noncircular. In an embodiment, the cross section is oval. The guide member 200 may have an outer surface 216. In an embodiment, the holding rod 150 and the guide member 200 may be formed as a single unit rather than as a two piece assembly as shown.

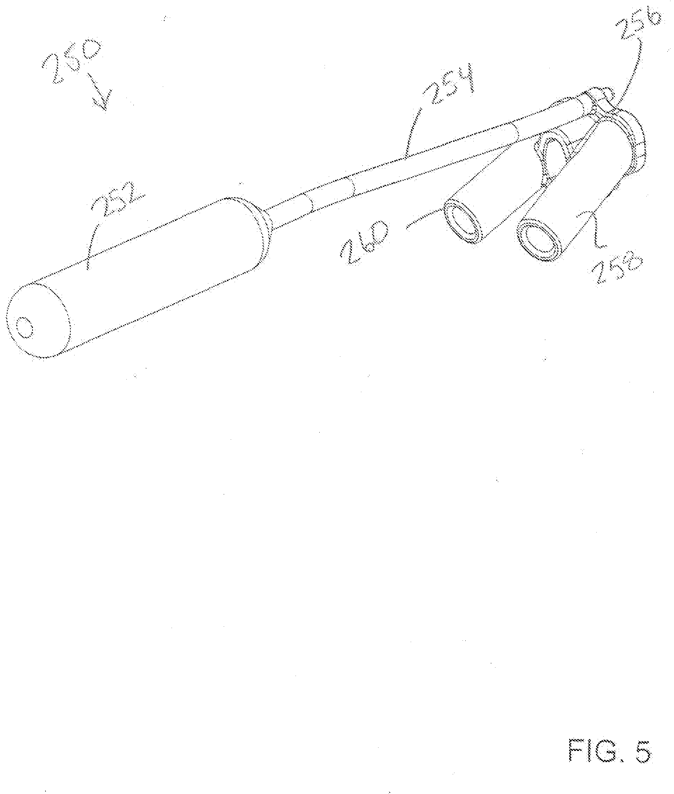

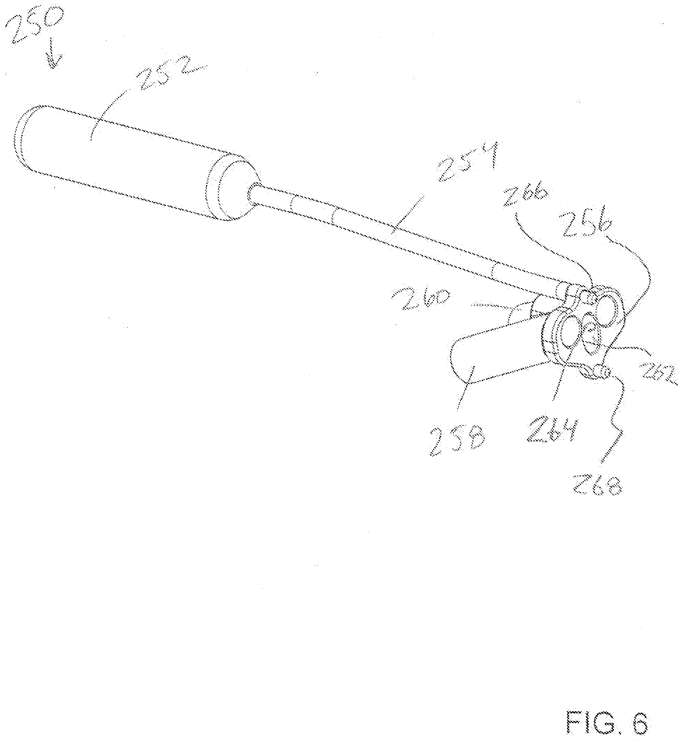

Referring now to FIGS. 5 and 6, there is depicted a drill guide 250 pursuant to an embodiment of the present disclosure. The drill guide 250 may comprise a handle 252. A shaft 254 may extend from the handle 252. A base member 256 may be attached to the distal end of the shaft 254. A first guide tube 258 and a second guide tube 260 may extend from the base member 256. It will be appreciated that the first guide tube 258 and the second guide tube 260 may each include a hollow passageway for allowing the passage of a drill bit and fasteners through the base member 256. Further, while the drill guide 250 is shown with two guide tubes, it will be appreciated that, pursuant to embodiments of the present disclosure, the drill guide 250 may comprise a single guide tube or any number of guide tubes.

As perhaps best seen in FIG. 6, an inner surface 262 of the base member 256 may define a guide hole 264. In an embodiment, the guide hole 264 is noncircular. In an embodiment, the guide hole 264 is oval. As will be explained in further detail hereinafter, the guide hole 264 may receive the guide member 200 to align and position the drill guide 250 over a disc space. Further, extending from the base member 256 may be a first guide post 266 and a second guide post 268. As will be explained in more detail hereinafter, the first guide post 266 and the second guide post 268 may align the drill guide 250 with respect to the interbody plate 170.

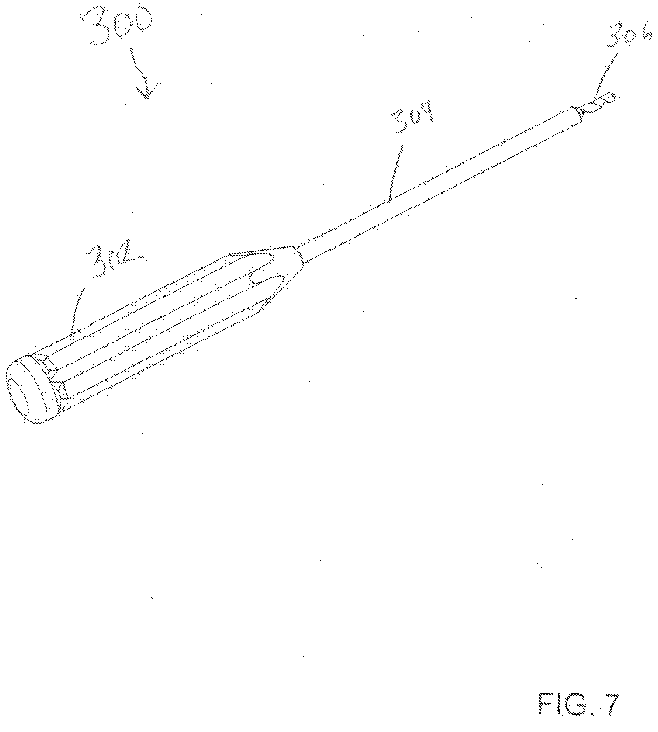

Referring now to FIG. 7, there is depicted a surgical drill 300 according to an embodiment of the present disclosure. The drill 300 may be a manually operated drill. In an embodiment, the drill 300 may be a powered surgical drill.

The drill 300 may comprise a handle 302. A shaft 304 may extend from the handle. It will be appreciated that the diameter of the shaft 304 may allow the shaft 304 to snugly fit in the passageways in the hollow tube guides 258 and 260 of the drill guide 250. Disposed on the end of the shaft 304 may be a drill bit 306. The drill bit 306 may be utilized to drill pilot holes in vertebrae as is known to one having ordinary skill.

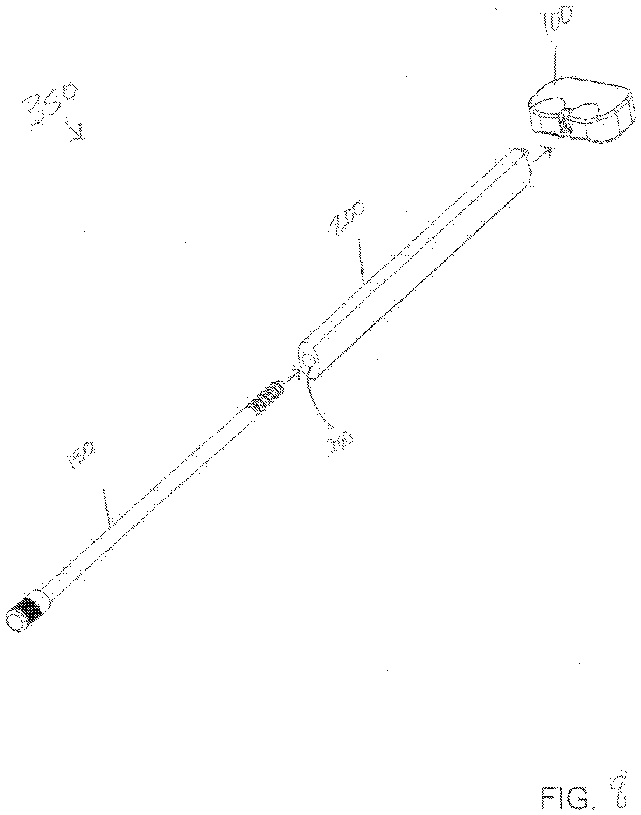

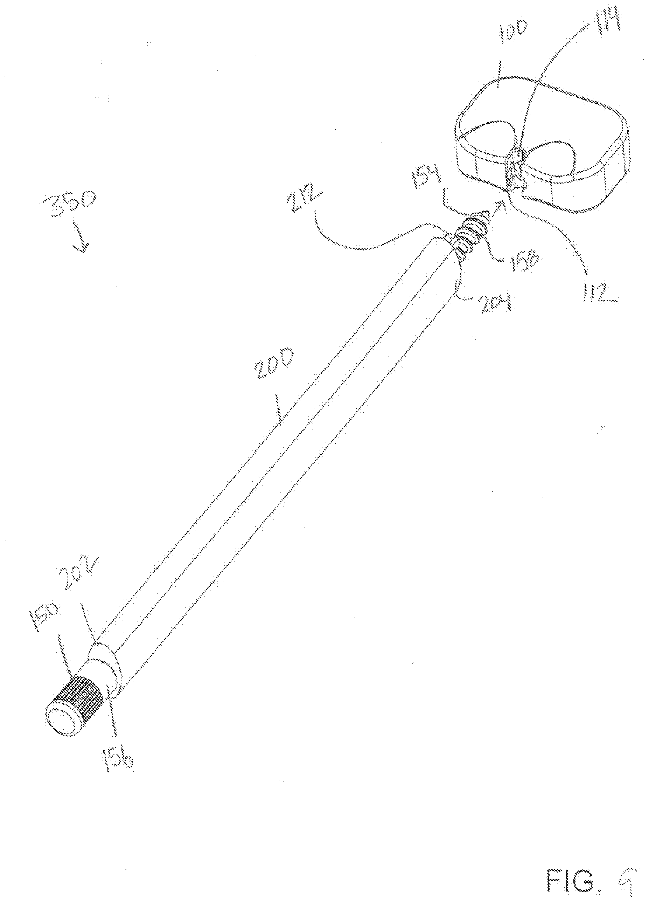

Referring now to FIGS. 8, 9, and 10, there is depicted an exploded view of a system 350 for spinal fixation. The system 350 may comprise the interbody cage 100, the guide member 200, and the holding rod 150. As perhaps best viewed in FIG. 9, the holding rod 150 is installed into the cannulation 210 of the guide member 200. The proximal end 202 of the guide member 200 may abut against the knob 156 of the holding rod 150. The length of the holding rod 150 may be sufficient such that the threads 158 on the distal end 154 extend from the distal end 204 of the guide member 200.

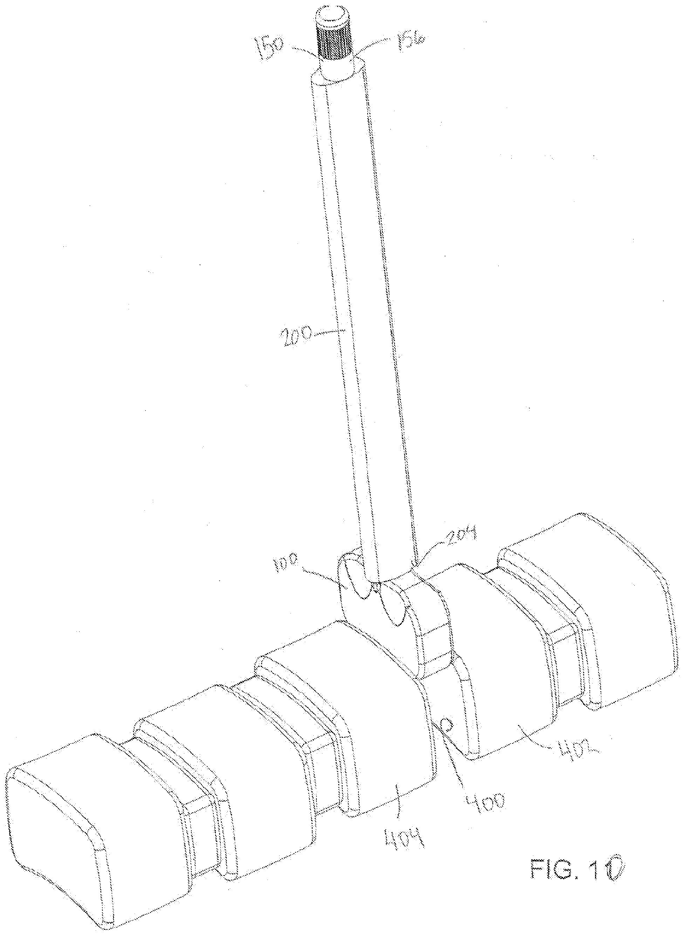

As perhaps best observed in FIG. 10, the distal end 154 of the holding rod 150 is installed into the threaded bore 112 of the interbody cage 100. The knob 156 may provide a grip for a surgeon to grasp and position the interbody cage 100. In addition, the knob 156 may receive hits from an impaction tool, such as a hammer, to impact the interbody cage 100. The distal end 204 of the guide member 200 may serve as a depth gauge when installing the interbody cage 100 by abutting against two vertebrae defining the disc space.

Further, the key members 212 and 214 extending from the distal end 204 of the guide member 200 may engage the key slot 114 of the interbody cage 100. It will be appreciated by those of ordinary skill that the key slot engagement positively locates the guide member 200 with respect to the interbody cage 100 and prevents the guide member 200 from moving or rotating about the holding rod 150 or its longitudinal axis 208.

Referring now to FIGS. 10-14, there is depicted a spinal fusion procedure according to an embodiment of the present disclosure. As previously described, the holding rod 150 may be utilized to removably secure the guide member 200 to the interbody cage 100 as observed in FIG. 10. This may be done prior to installing the interbody cage 100 into the disc space.

A disc space 400 defined between a first vertebra 402 and a second vertebra 404 may be prepared by removing the natural disc as is known to those having ordinary skill. Next, the interbody cage 100 may be positioned into the disc space 400 by a surgeon using the holding rod 150 having the guide member 200 disposed thereon. The interbody cage 100 may be impacted into the disc space 400. Once the interbody cage 100 has been installed in the disc space 400, it may be positioned using the holding rod 150. The depth of the cage 100 in the disc space may be determined by the distal end 204 of the guide member 200, which may abut against the first vertebra 402 and the second vertebra 404. It will be appreciated that the guide member 200 may extend outside of the body of the patient when attached to the interbody cage 100.

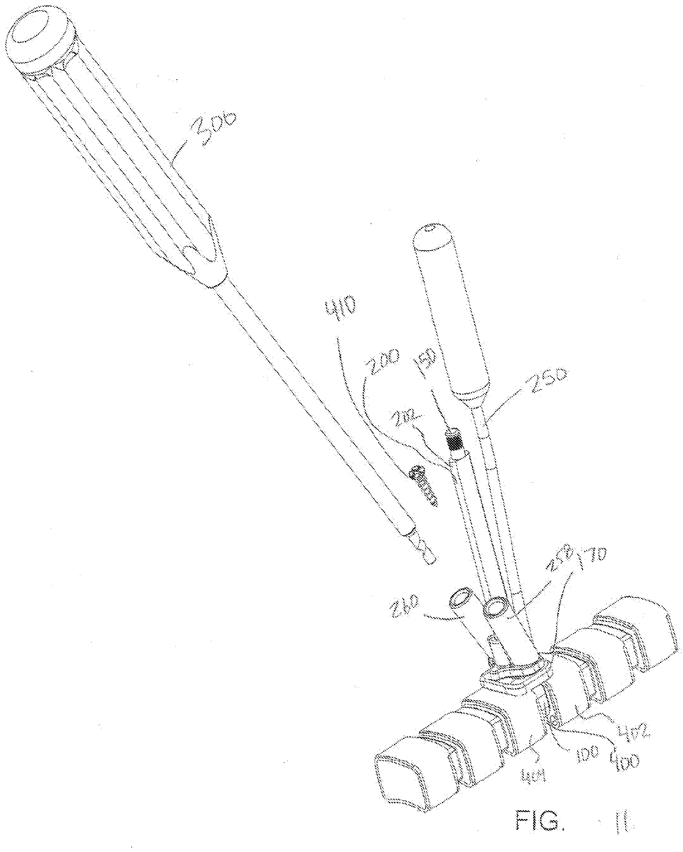

As seen in FIG. 11, with the interbody cage 100 positioned in the disc space 400, the interbody plate 170 is aligned and positioned over the disc space 400 using the guide member 200. In particular, the proximal end 202 of the guide member 200 is installed into the guide hole 178 of the interbody plate 170. The plate 170 is then slid down the length of guide member 200 into position over the disc space 400.

The guide member 200 may position and align the interbody plate 170 above the interbody cage 100. In particular, the inner surface 176 of the plate 170 that defines the guide hole 178 may engage the outer surface 216 of the guide member 200. It will be appreciated that the noncircular nature of the cross section of the guide member 200 prevents the plate 170 from rotating or otherwise becoming misaligned over the disc space 400.

Once the interbody plate 170 is positioned over the disc space 400, the drill guide 250 may be installed onto the guide member 200 and positioned over the interbody plate 170. In particular, the guide hole 264 of the drill guide 250 is installed onto the proximal end 202 of guide member 200 and slid into position over the interbody plate 170. At this point, the first guide post 266 and the second guide post 268 of the base member 256 of the drill guide 250 may engage the pair of guide holes 182A and 182B of the interbody plate 170 to ensure that the drill guide 250 is properly positioned with respect to the plate 170 and the cage 100.

In an embodiment, the drill guide 250 and the plate 170 may be preassembled such that they can be installed as one unit over the guide member 200. Various methods of securing the drill guide 250 and the plate 170 together are envisioned and fall within the scope of the present disclosure. In an embodiment, the guide posts 266 and 268 may have a press or snap fit into the guide holes 182A and 182B of the plate 170. In an embodiment, the guide posts 266 and 268 may threadably engage the guide holes 182A and 182B of the plate 170. In an embodiment, the drill guide 250 and the plate 170 may be magnetically coupled. Thus, the present disclosure contemplates various engagements means to secure the drill guide 250 and the plate 170 together such that they can be inserted together. Once the fasteners are installed to secure the plate 170, as explained below, the drill guide 250 may be detached from the plate 170 to allow the drill guide 250 to be removed.

Once the drill guide 250 is in place and locked with the plate 170, the drill 300 may be utilized to drill pilot holes in the vertebrae 402 and 404. In particular, with the guide tubes 258 and 260 of the drill guide 250 aligned over the first set of holes 180A, 180B, the drill 300 may be utilized to drill two pilot holes in the vertebra 402. It will be appreciated that the drill bit 306 passes through the holes 180A and 180B in the plate 170. Further, as the diameter of the shaft 304 of the drill 300 is just slightly smaller than the diameter of the guide tubes 258 and 260 of the drill guide 250, the bit 306 will be properly aligned. Once the pilot holes have been created, and before the drill guide 250 is removed, fasteners 410 may be installed into the guide tubes 258 and 260 to secure the plate 170 to the first vertebra 402.

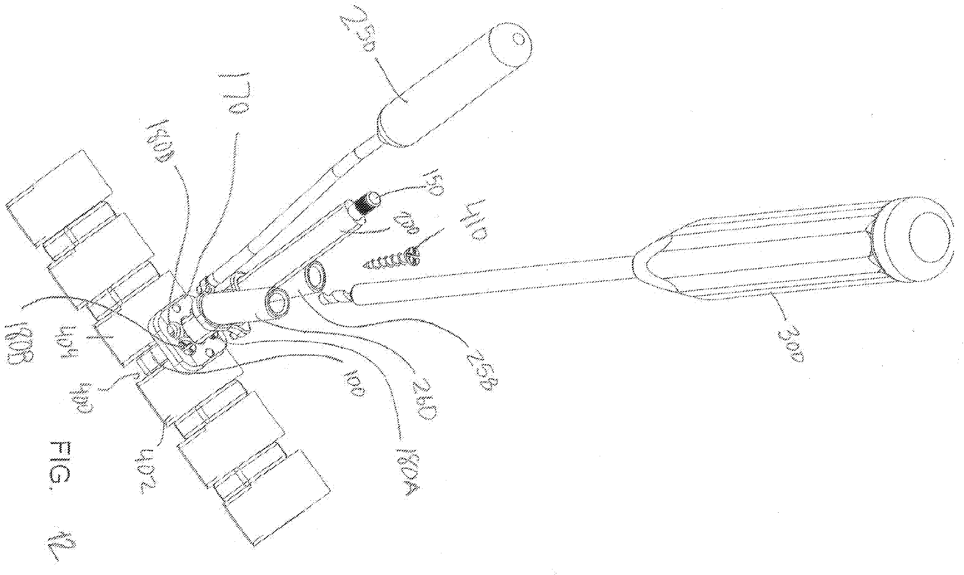

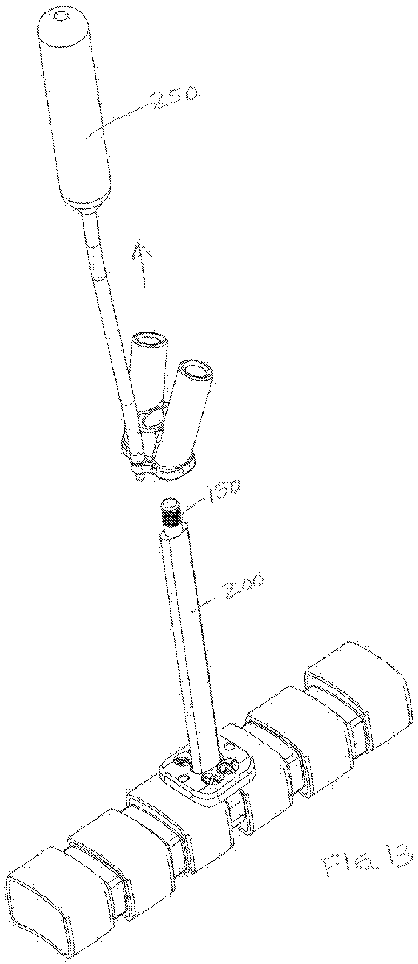

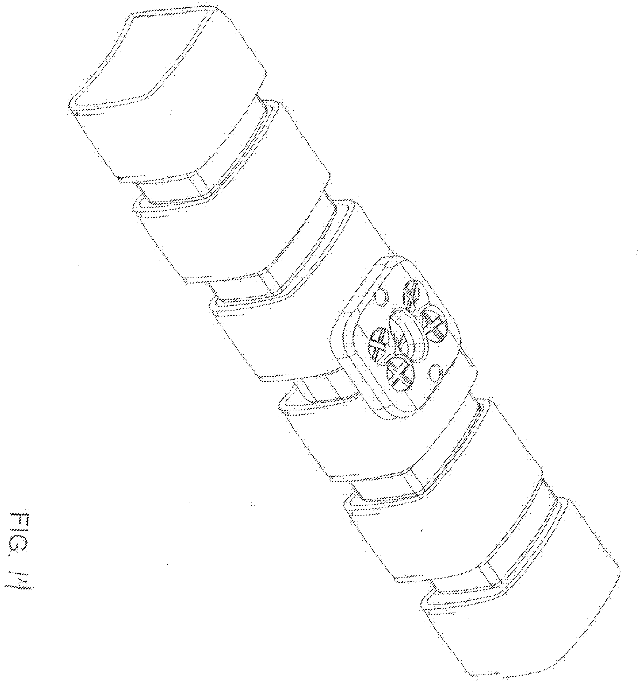

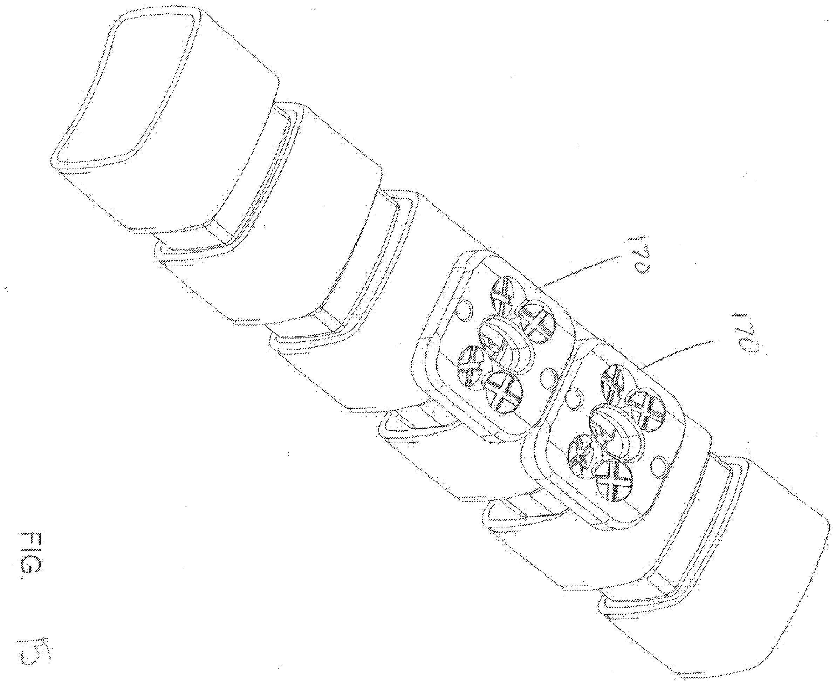

As seen in FIG. 12, once the fasteners 410 have been installed into the first set of holes 180A, 180B in the first vertebra 402, the drill guide 250 may be removed from the guide member 200, reoriented by 180 degrees, and then reinstalled onto the guide member 200. The drill 300 may then be utilized to drill pilot holes in the vertebra 404. Again fasteners 410 may be utilized to secure the interbody plate 170 to the vertebra 404 through the second set of holes 180C, 180D in the plate 170. With the interbody plate 170 secured, the drill guide 250 and the elongated guide member 200 may be removed as shown in FIGS. 13 and 14. In particular, to remove the elongated guide member 200, the threads 158 on the distal end 154 of the guide member 200 may be unscrewed from the threaded bore 112 of the interbody cage 100. As shown in FIG. 15, multiple interbody plates 170 may be utilized to fuse several levels of vertebrae of a patient.

It will be appreciated that although the engagement that removably secures the guide member 200 to the interbody cage 100 has been described herein as a threaded engagement between the holding rod 150 and the threaded bore 112, that any engagement that removably secures the guide member 200 to the interbody cage 100 falls within the scope of the present disclosure. Further, it will be appreciated that the grooves in the superior surface 104 and the inferior surface 105 of the body member 102 of the interbody cage 100 allow the fasteners utilized to secure the interbody plate 170 to pass within the grooves.

It will be appreciated that it is a distinct advantage of the present disclosure to accurately position the interbody plate 170 above a vertebral gap. This accurate positioning of the plate 170 may allow the length of the interbody plate 170 to be kept to a minimum, which may allow the use of multiple plates 170 at multiple adjacent levels in the spine as shown in FIG. 15. This feature may have biomechanical advantages and improve fusion rates over single long plates. It also means that where access is limited, such as in minimally invasive procedures, the more easily small plates can be selected and positioned without fear the screws will damage or move a cage installed between the vertebral space.

Further, it will be appreciated that using the plate 170 would mean that the bone fasteners may cut into the cage 100 without the grooves in the superior and inferior surfaces of the cage 100 (see FIG. 1). In an embodiment, the grooves are positioned to align with the shortest plates possible for any given size of cage so that even with a very short plate, the fasteners will not cut the cage.

In an embodiment, the plate 170 may be utilized as a buttress plate that is only secured to a single vertebra and whose sole function is prevent back out of a cage. It will be appreciated that this may be useful where a second stage adjustment of the relative position of the vertebrae is desired and would be limited by having fasteners installed into both vertebrae. Such a buttress plate may have one or two screws, but again correct alignment and ease of placement would be helpful using the concepts of the present invention.

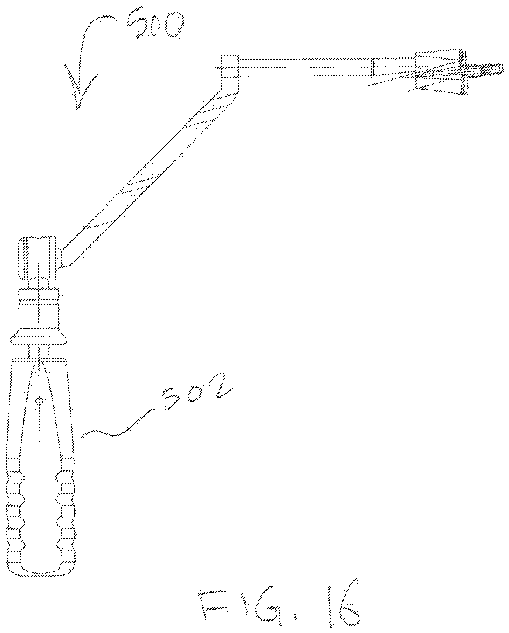

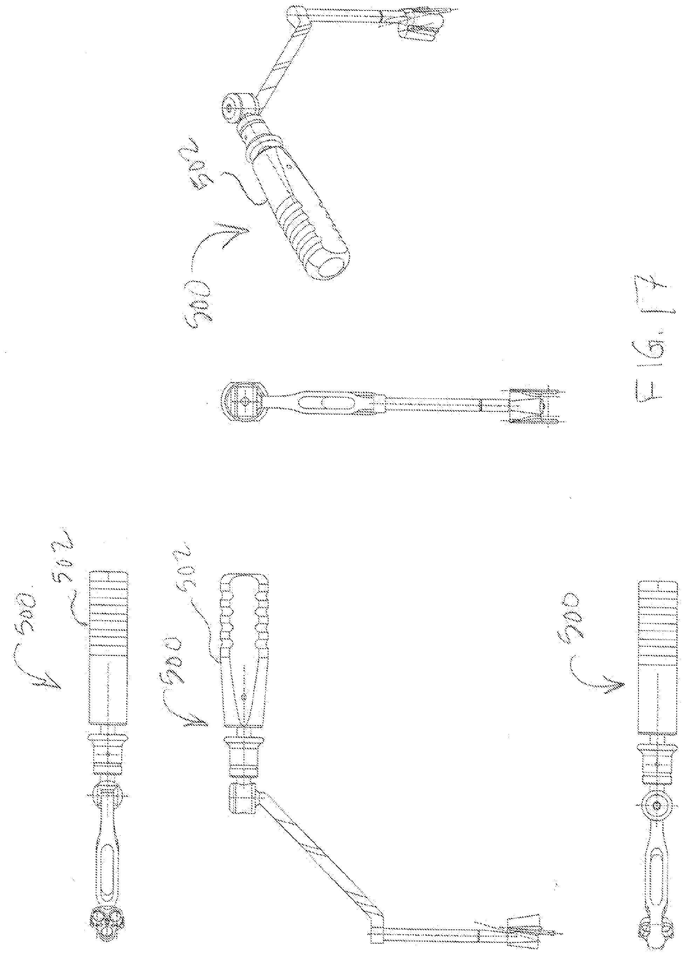

FIGS. 16-17 illustrate a drill guide 500 having a handle 502 that has a hollow section rather than a curved shape, which can allow a long drill to pass into the drill guide without fouling or soiling the handle.

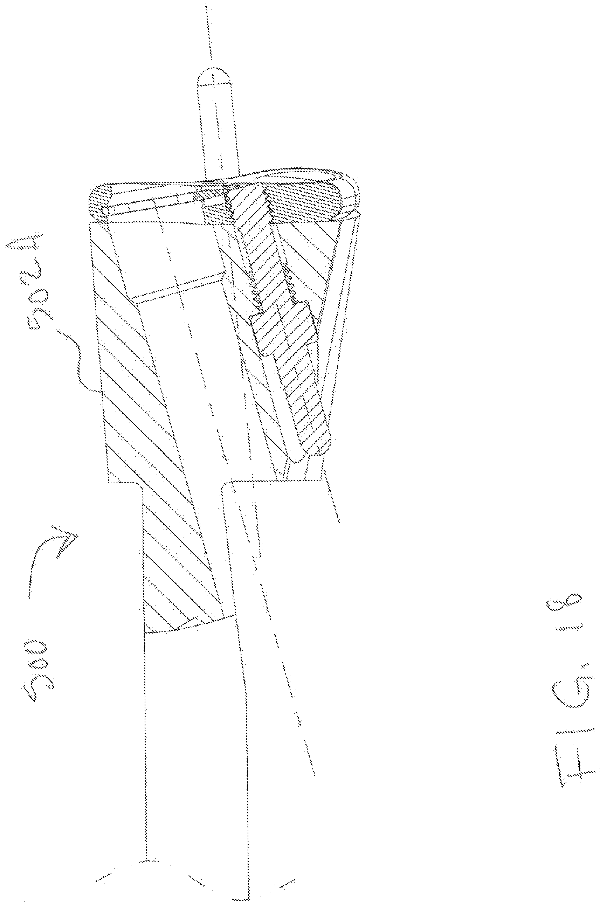

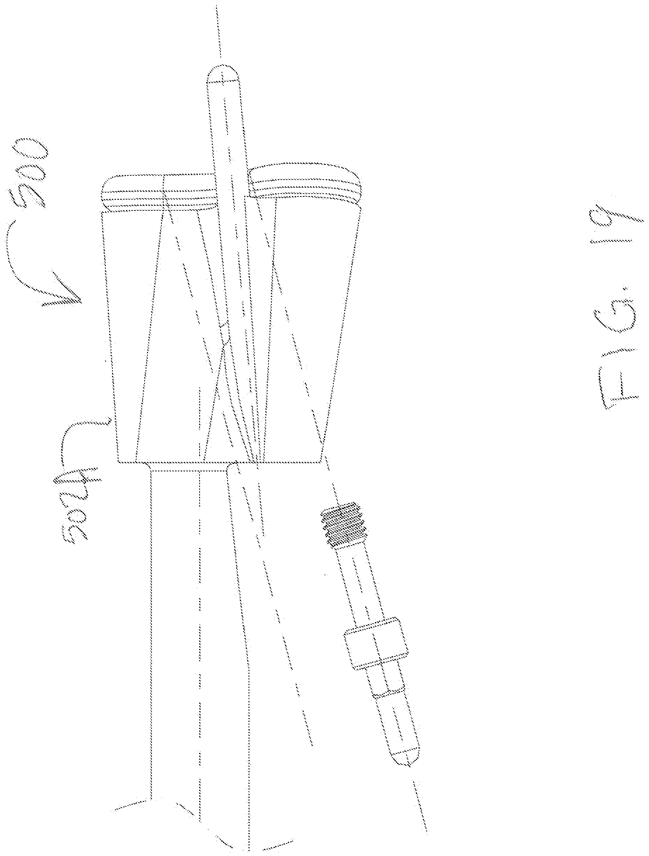

FIGS. 18-19 illustrate section 502A of the drill guide 500, and detail shown thereby.

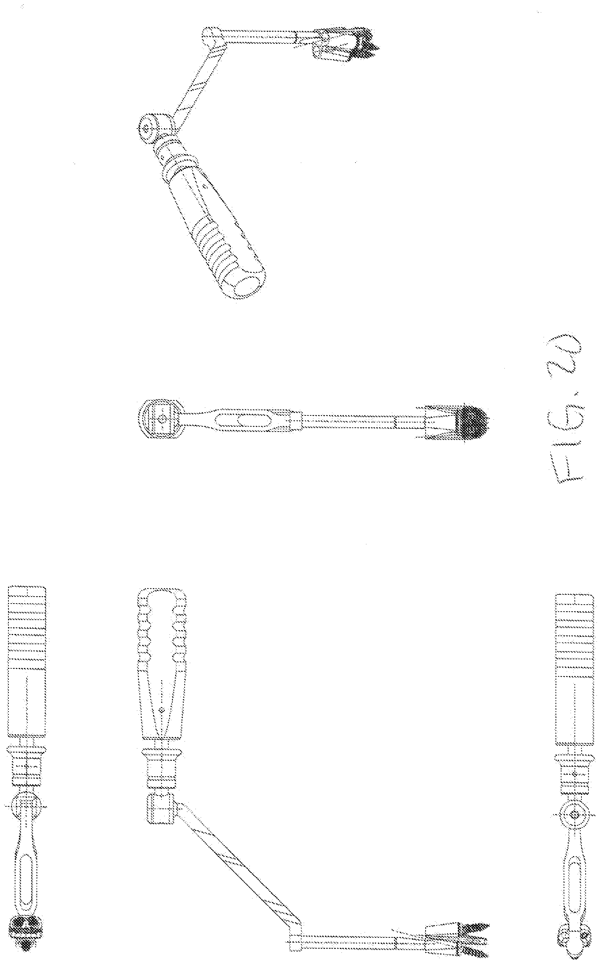

FIG. 20 illustrates further detail of the matter shown in FIGS. 16-19.

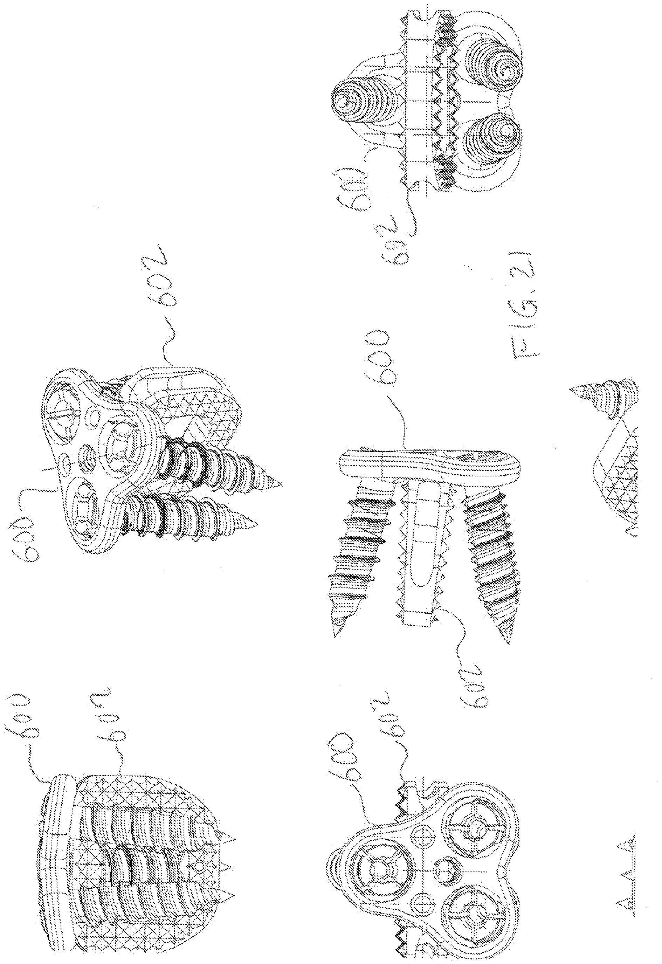

FIG. 21 illustrates positioning plate 600, which may extend downward from a drill guide, such as drill guide 500 of FIGS. 16-20 or other suitable drill guide. The plate 600 may be the same height as cage 602, and mya thus fit snugly into a disc space. Multiple plates 600 may be utilized to grip both lateral side of a cage 602, the plates 600 being of a similar height and shape of cage 602, and thus the plates 600 may also help position or locate a drill guide.

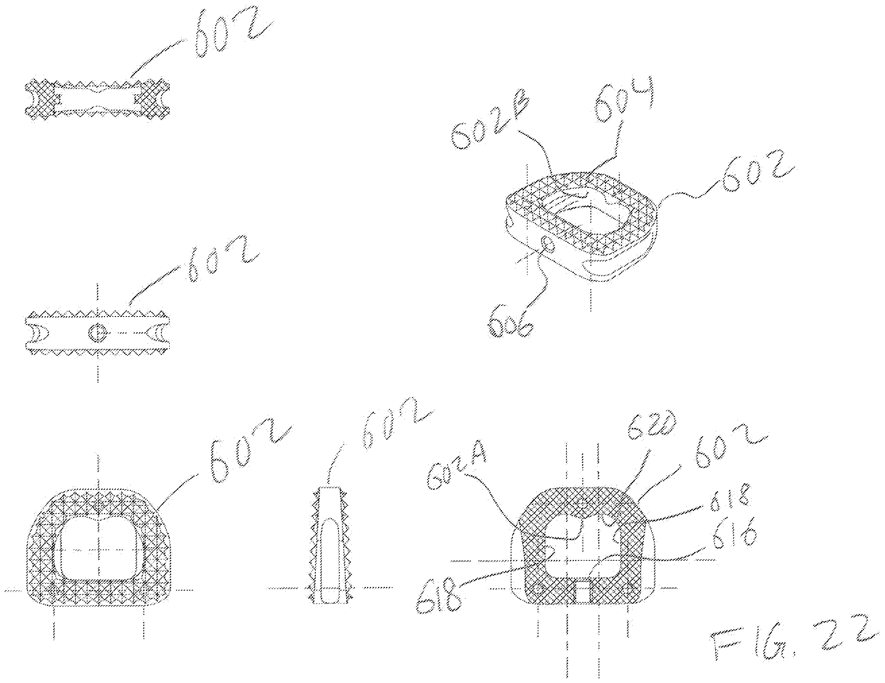

FIG. 22 shows that cage 602 is hollow, may have fixation surface 604 including spikes as shown or other suitable fixation structure, inserter hole 606. Cage 602 have a nub 602A formed by internal sidewalls 602B, making a central opening which may have a shape that may have a bottom straight section 616, opposing straight side sections 618, and upper section 620 having dual opposing concave curves intercoupled together at the nub 602A.

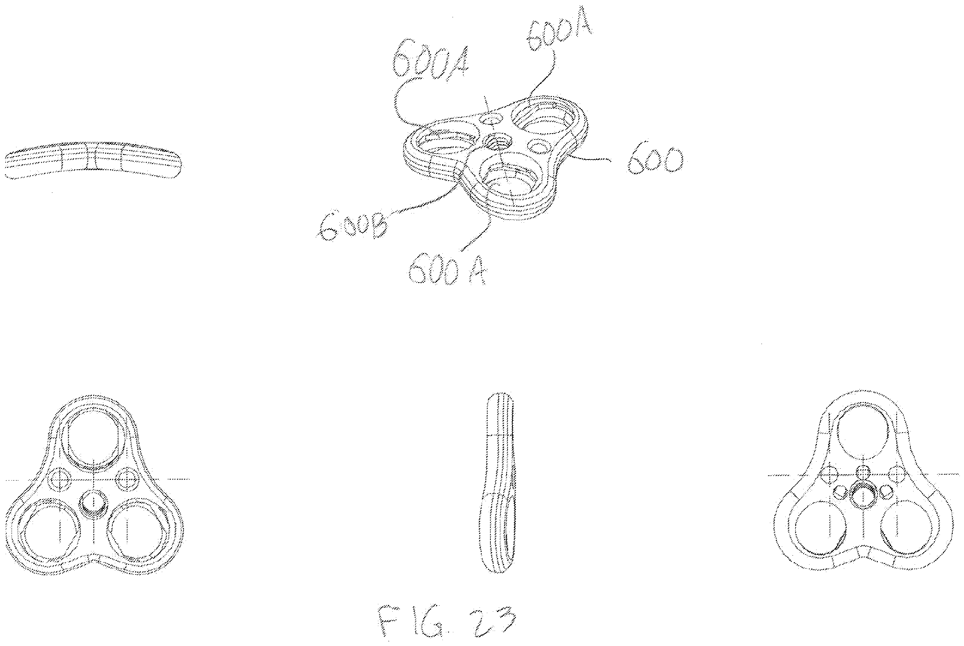

FIG. 23 shows plate 600 having fastener triple holes 600A, and central guide hole 600B.

Those having ordinary skill in the relevant art will appreciate the advantages provided by the features of the present disclosure. For example, it is a feature of the present disclosure to provide a spinal plate selection and positioning system. Another feature of the present disclosure is to provide a guide member that removably attaches to an interbody cage, the guide member aligning and positioning an interbody plate and drill guide. It is a further feature of the present disclosure, in accordance with one aspect thereof, to provide an interbody cage with grooves in its superior and inferior surfaces for allowing passage of fasteners utilized to secure an interbody plate.

In the foregoing Detailed Description, various features of the present disclosure are grouped together in a single embodiment for the purpose of streamlining the disclosure. This method of disclosure is not to be interpreted as reflecting an intention that the claimed disclosure requires more features than are expressly recited in each claim. Rather, as the following claims reflect, inventive aspects lie in less than all features of a single foregoing disclosed embodiment.

It is to be understood that the above-described arrangements are only illustrative of the application of the principles of the present disclosure. Numerous modifications and alternative arrangements may be devised by those skilled in the art without departing from the scope of the present disclosure and the appended claims are intended to cover such modifications and arrangements. Thus, while the present disclosure has been shown in the drawings and described above with particularity and detail, it will be apparent to those of ordinary skill in the art that numerous modifications, including, but not limited to, variations in size, materials, shape, form, function and manner of operation, assembly and use may be made without departing from the principles and concepts set forth herein.

## Claims

1. A system for fusing a first vertebra (402) and a second vertebra (404) of a spinal column, the first vertebra and second vertebra defining a disc space (400), said system comprising:
an interbody cage (100) having a proximal end with a proximal surface (108) and a distal end with a distal surface (110), the interbody cage configured and dimensioned to fit within the disc space (400);
an elongated guide member (200) removably attachable to the interbody cage (100), the elongated guide member extending along a longitudinal axis (208) from a proximal end (202) to a distal end (204), the elongated guide member having an outer surface (216); and
an interbody plate (170) having an inner surface (176) defining a guide hole (178), said guide hole configured and dimensioned to receive the elongated guide member (200) to thereby align and position said interbody plate (170) over the disc space (400);
wherein the inner surface (176) of the interbody plate (170) engages the outer surface (216) of the elongated guide member (200) to prevent the interbody plate (170) from rotating about the longitudinal axis (208) of the elongated guide member (200);
the interbody cage (100) also includes a first groove (120) and a second groove (122), each of the first groove and the second groove extending from the proximal surface (108) toward the distal surface (110); and
**characterised in that** the elongated guide member (200) has a non-circular cross-section in a plane perpendicular to its longitudinal axis (208) and the guide hole (178) of the interbody plate (170) has a non-circular cross-section complementary to the non-circular cross-section of the elongated guide member (200).

2. The system of claim 1, wherein the interbody cage (100) comprises:
a body member (102), the body member having a superior surface (104) and an inferior surface (105);
the body member (102) further having a sidewall (106) extending between the superior surface (104) and the inferior surface (105), the sidewall circumscribing the body member;
a first set of grooves (120, 122) formed in the superior surface (104), the first set of grooves extending from the proximal end towards the distal end; and
a second set of grooves (120, 122) formed in the inferior surface (105), the second set of grooves extending from the proximal end towards the distal end.

3. The system of claim 1, wherein the interbody plate (170) comprises a plurality of holes (180A, 180B, 180C, 180D) for receiving fasteners such that the interbody plate is securable to the first vertebra (402) and the second vertebra (404).

4. The system of claim 3, wherein the holes comprise a first set of holes (180A, 180B) and a second set of holes (180C, 180D).

5. The system of claim 4, wherein one of the first set of holes (180A, 180B) is disposed on either side of the guide hole (178) and one of the second set of holes (180C, 180D) is disposed on either side of the guide hole (178).

6. The system of claim 5, wherein each of the first set of holes and each of the second set of holes comprises an axis, wherein the axis of each of the first set of holes and the second set of holes forms an acute angle with a plane containing an axis of the guide hole.

7. The system of claim 1, wherein the proximal end of the interbody cage (100) further comprises a threaded bore (112).

8. The system of claim 7, further comprising a holding rod (150) having a threaded end (158), said threaded end configured and dimensioned to engage the threaded bore (112) of the interbody cage (100).

9. The system of claim 8, wherein the elongated guide member (200) comprises a cannulation (210) along its longitudinal axis (208), and the holding rod (150) is configured and dimensioned to pass through the cannulation (210) such that the threaded end (158) of the holding rod (150) can be installed in the threaded bore (112) of the interbody cage (100).

10. The system of claim 9, wherein the proximal end of the interbody cage (100) comprises a key slot (114), and wherein a distal end (204) of the elongated guide member (200) comprises a key (212, 214), and wherein said key of the elongated guide member is removably installable in the key slot (114) of the interbody cage (100) such that the elongated guide member is prevented from rotating about its longitudinal axis (208).

11. The system of claim 1, further comprising a key (212, 214) and a key slot (114) to prevent the elongated guide member (200) from rotating about its longitudinal axis (208).

12. The system of claim 1, further comprising:
a drill guide (250) having an inner surface (262) defining a guide hole (264), said guide hole configured and dimensioned to receive the elongated guide member (200) such that said drill guide (250) can slide down the elongated guide member into position over the interbody cage (100);
wherein the inner surface (262) of the drill guide (250) engages the outer surface (216) of the elongated guide member (200) to prevent the drill guide from rotating about the longitudinal axis (208) of the elongated guide member.

13. The system of claim 12, wherein the drill guide comprises a plurality of bit guide tubes (258, 260) for receiving a drill bit.

14. The system of claim 12, wherein the plurality of bit guide tubes (258, 260) are angled.

## Patentansprüche

1. System zum Fusionieren eines ersten Wirbels (402) und eines zweiten Wirbels (404) einer Wirbelsäule, wobei der erste Wirbel und der zweite Wirbel einen Bandscheibenraum (400) definieren, wobei das System umfasst:
einen Zwischenkörperkäfig (100) mit einem proximalen Ende mit einer proximalen Fläche (108) und einem distalen Ende mit einer distalen Fläche (110), wobei der Zwischenkörperkäfig dafür konfiguriert und dimensioniert ist, in den Bandscheibenraum (400) zu passen;
ein längliches Führungselement (200), das am Zwischenkörperkäfig (100) abnehmbar befestigbar ist, wobei sich längliche Führungselement entlang einer Längsachse (208) von einem proximalen Ende (202) zu einem distalen Ende (204) erstreckt, wobei das längliche Führungselement eine Außenfläche (216) aufweist; und
eine Zwischenkörperplatte (170) mit einer ein Führungsloch (178) definierenden Innenfläche (176), wobei das Führungsloch dafür konfiguriert und dimensioniert ist, das längliche Führungselement (200) aufzunehmen, um dadurch die Zwischenkörperplatte (170) über dem Bandscheibenraum (400) auszurichten und zu positionieren;
wobei die Innenfläche (176) der Zwischenkörperplatte (170) in die Außenfläche (216) des länglichen Führungselements (200) eingreift, um die Zwischenkörperplatte (170) am Drehen um die Längsachse (208) des länglichen Führungselements (200) zu hindern;
der Zwischenkörperkäfig (100) auch eine erste Rille (120) und eine zweite Rille (122) beinhaltet, wobei sich jede der ersten Rille und der zweiten Rille von der proximalen Fläche (108) hin zur distalen Fläche (110) erstreckt; und
**dadurch gekennzeichnet, dass** das längliche Führungselement (200) einen nicht kreisförmigen Querschnitt in einer Ebene senkrecht zu seiner Längsachse (208) aufweist und das Führungsloch (178) der Zwischenkörperplatte (170) einen nicht kreisförmigen Querschnitt komplementär zum nicht kreisförmigen Querschnitt des länglichen Führungselements (200) aufweist.

2. System nach Anspruch 1, wobei der Zwischenkörperkäfig (100) umfasst:
ein Körperelement (102), wobei das Körperelement eine obere Fläche (104) und eine untere Fläche (105) aufweist;
das Körperelement (102) ferner eine Seitenwand (106) mit Erstreckung zwischen der oberen Fläche (104) und der unteren Fläche (105) aufweist, wobei die Seitenwand das Körperelement umschreibt;
einen ersten Satz von in der oberen Fläche (104) gebildeten Rillen (120, 122), wobei sich der erste Satz von Rillen vom proximalen Ende hin zum distalen Ende erstreckt; und
einen zweiten Satz von in der unteren Fläche (105) gebildeten Rillen (120, 122), wobei sich der zweite Satz von Rillen vom proximalen Ende hin zum distalen Ende erstreckt.

3. System nach Anspruch 1, wobei die Zwischenkörperplatte (170) eine Mehrzahl von Löchern (180A, 180B, 180C, 180D) zum Aufnehmen von Befestigungselementen umfasst, sodass die Zwischenkörperplatte an dem ersten Wirbel (402) und dem zweiten Wirbel (404) sicherbar ist.

4. System nach Anspruch 3, wobei die Löcher einen ersten Satz von Löchern (180A, 180B) und einen zweiten Satz von Löchern (180C, 180D) umfassen.

5. System nach Anspruch 4, wobei eines des ersten Satzes von Löchern (180A, 180B) beiderseits des Führungslochs (178) angeordnet ist und eines des zweiten Satzes von Löchern (180C, 180D) beiderseits des Führungslochs (178) angeordnet ist.

6. System nach Anspruch 5, wobei jedes des ersten Satzes von Löchern und jedes des zweiten Satzes von Löchern eine Achse umfasst, wobei die Achse jedes des ersten Satzes von Löchern und des zweiten Satzes von Löchern einen spitzen Winkel zu einer eine Achse des Führungslochs enthaltenden Ebene bildet.

7. System nach Anspruch 1, wobei das proximale Ende des Zwischenkörperkäfigs (100) ferner eine Gewindebohrung (112) umfasst.

8. System nach Anspruch 7, ferner umfassend eine Haltestange (150) mit einem Gewindeende (158), wobei das Gewindeende dafür konfiguriert und dimensioniert ist, in die Gewindebohrung (112) des Zwischenkörperkäfigs (100) einzugreifen.

9. System nach Anspruch 8, wobei das längliche Führungselement (200) eine Kanülierung (210) entlang seiner Längsachse (208) umfasst und die Haltestange (150) dafür konfiguriert und dimensioniert ist, die Kanülierung (210) zu durchlaufen, sodass das Gewindeende (158) der Haltestange (150) in der Gewindebohrung (112) des Zwischenkörperkäfigs (100) installiert werden kann.

10. System nach Anspruch 9, wobei das proximale Ende des Zwischenkörperkäfigs (100) einen Keilschlitz (114) umfasst und wobei ein distales Ende (204) des länglichen Führungselements (200) einen Keil (212, 214) umfasst, und wobei der Keil des länglichen Führungselements im Keilschlitz (114) des Zwischenkörperkäfigs (100) abnehmbar installierbar ist, sodass das längliche Führungselement am Drehen um seine Längsachse (208) gehindert wird.

11. System nach Anspruch 1, ferner umfassend einen Keil (212, 214) und einen Keilschlitz (114), um das längliche Führungselement (200) am Drehen um seine Längsachse (208) zu hindern.

12. System nach Anspruch 1, ferner umfassend:
eine Bohrerführung (250) mit einer ein Führungsloch (264) definierenden Innenfläche (262), wobei das Führungsloch dafür konfiguriert und dimensioniert ist, das längliche Führungselement (200) aufzunehmen, sodass die Bohrerführung (250) das längliche Führungselement hinunter in Position über dem Zwischenkörperkäfig (100) gleiten kann;
wobei die Innenfläche (262) der Bohrerführung (250) in die Außenfläche (216) des länglichen Führungselements (200) eingreift, um die Bohrerführung am Drehen um die Längsachse (208) des länglichen Führungselements zu hindern.

13. System nach Anspruch 12, wobei die Bohrerführung eine Mehrzahl von Aufsatzführungsrohren (258, 260) zum Aufnehmen eines Bohraufsatzes umfasst.

14. System nach Anspruch 12, wobei die Mehrzahl von Aufsatzführungsrohren (258, 260) angewinkelt ist.

## Revendications

1. Système de fusion d'une première vertèbre (402) et d'une deuxième vertèbre (404) d'une colonne vertébrale, la première vertèbre et la deuxième vertèbre définissant un espace discal (400), ledit système comprenant :
une cage intersomatique (100) ayant une extrémité proximale avec une surface proximale (108) et une extrémité distale avec une surface distale (110), la cage intersomatique étant configurée et dimensionnée pour s'adapter dans l'espace discal (400) ;
un élément de guidage allongé (200) pouvant être fixé de manière amovible à la cage intersomatique (100), l'élément de guidage allongé s'étendant sur un axe longitudinal (208) depuis une extrémité proximale (202) jusqu'à une extrémité distale (204), l'élément de guidage allongé ayant une surface extérieure (216) ; et
une plaque intersomatique (170) ayant une surface interne (176) définissant un trou de guidage (178), ledit trou de guidage étant configuré et dimensionné pour recevoir l'élément de guidage allongé (200) de manière à aligner et à positionner ladite plaque intersomatique (170) sur l'espace discal (400) ;
où la surface interne (176) de la plaque intersomatique (170) vient en prise avec la surface externe (216) de l'élément de guidage allongé (200) afin d'empêcher la plaque intersomatique (170) de tourner autour de l'axe longitudinal (208) de l'élément de guidage allongé (200) ;
la cage intersomatique (100) incluant également une première gorge (120) et une deuxième gorge (122), la première gorge et la deuxième gorge s'étendant de la surface proximale (108) vers la surface distale (110) ; et
**caractérisée en ce que** l'élément de guidage allongé (200) comporte une section transversale non circulaire dans un plan perpendiculaire à son axe longitudinal (208) et le trou de guidage (178) de la plaque intersomatique (170) comporte une section transversale non circulaire venant compléter la section transversale non circulaire de l'élément de guidage allongé (200).

2. Le système selon la revendication 1, dans lequel la cage intersomatique (100) comprend :
un élément de corps (102), l'élément de corps ayant une surface supérieure (104) et une surface inférieure (105) ;
l'élément de corps (102) ayant en outre une paroi latérale (106) s'étendant entre la surface supérieure (104) et la surface inférieure (105), la paroi latérale entourant l'élément de corps ;
un premier ensemble de gorges formées (120, 122) dans la surface supérieure (104), le premier ensemble de gorges s'étendant de l'extrémité proximale vers l'extrémité distale ; et
un deuxième ensemble de gorges (120, 122) formées dans la surface inférieure (105), le deuxième ensemble de gorges s'étendant de l'extrémité proximale vers l'extrémité distale.

3. Système selon la revendication 1, dans lequel la plaque intersomatique (170) comprend une pluralité de trous (180A, 180B, 180C, 180D) pour recevoir des attaches de sorte que la plaque intersomatique puisse être fixée à la première vertèbre (402) et à la seconde vertèbre (404).

4. Système selon la revendication 3, dans lequel les trous comprennent un premier ensemble de trous (180A, 180B) et un deuxième ensemble de trous (180C, 180D).

5. Système selon la revendication 4, dans lequel l'un parmi le premier ensemble de trous (180A, 180B) est disposé de chaque côté du trou de guidage (178) et l'un parmi le deuxième ensemble de trous (180C, 180D) est disposé de chaque côté du trou de guidage (178).

6. Système selon la revendication 5, dans lequel chacun parmi le premier groupe de trous et chacun parmi le deuxième groupe de trous comprend un axe, où l'axe de chacun parmi le premier groupe de trous et le deuxième groupe de trous forme un angle aigu avec un plan contenant un axe du trou de guidage.

7. Système selon la revendication 1, dans lequel l'extrémité proximale de la cage intersomatique (100) comprend en outre un alésage fileté (112).

8. Système la revendication 7, comprenant en outre une tige de maintien (150) ayant une extrémité filetée (158), ladite extrémité filetée étant configurée et dimensionnée pour venir en prise avec l'alésage fileté (112) de la cage intersomatique (100).

9. Système selon la revendication 8, dans lequel l'élément de guidage allongé (200) comprend une canulation (210) le long de son axe longitudinal (208) et la tige de maintien(150) est configurée et dimensionnée pour passer à travers la canulation (210) de sorte que l'extrémité filetée (158) de la tige de maintien (150) puisse être installée dans l'alésage fileté (112) de la cage intersomatique (100).

10. Système selon la revendication 9, dans lequel l'extrémité proximale de la cage intersomatique (100) comprend une fente pour clé (114), et dans lequel une extrémité distale (204) de l'élément de guidage allongé (200) comprend une clé (212, 214), et dans lequel ladite clé de l'élément de guidage allongé peut être installée de manière amovible dans la fente de clé (114) de la cage intersomatique (100) de sorte que l'élément de guidage allongé ne puisse pas tourner autour de son axe longitudinal (208).

11. Système selon la revendication 1, comprenant en outre une clé (212, 214) et une fente pour clé (114) pour empêcher l'élément de guidage allongé (200) de tourner autour de son axe longitudinal (208).

12. Système selon la revendication 1, comprenant en outre :
un guide de foret (250) ayant une surface interne (262) définissant un trou de guidage (264), ledit trou de guidage étant configuré et dimensionné pour recevoir l'élément de guidage allongé (200) de sorte que ledit guide de foret (250) puisse coulisser le long de l'élément de guidage allongé pour se positionner sur la cage intersomatique (100) ;
où la surface interne (262) du guide de foret (250) vient en prise avec la surface externe (216) de l'élément de guidage allongé (200) pour empêcher le guide de foret de tourner autour de l'axe longitudinal (208) de l'élément de guidage allongé.

13. Système selon la revendication 12, dans lequel le guide de foret comprend une pluralité de tubes de guidage de trépan (258, 260) destinés à recevoir un trépan.

14. Système selon la revendication 12, dans lequel la pluralité de tubes de guidage de trépan (258, 260) est inclinée.
